# EUROPEAN PATENT SPECIFICATION

(11) **EP 4 554 537 B1**
(45) Date of publication and mention of the grant of the patent: **25.11.2026**
(21) Application number: 23755189.0
(22) Date of filing: 10.07.2023
(51) Int. Cl.: A61F 9/007, A61N 7/00, A61B 17/225, A61B 18/20, A61B 90/00, A61B 90/30, A61B 17/22

(54) **PULSATILE SUCTION RING FOR VISUALIZING AND TREATING DRAINAGE PATHWAYS OF THE EYE**
PULSIERENDER ABSAUGRING ZUR VISUALISIERUNG UND BEHANDLUNG VON DRAINAGEPFADEN DES AUGES
ANNEAU D'ASPIRATION PULSATILE POUR VISUALISER ET TRAITER DES VOIES DE DRAINAGE DE L'OEIL

(30) Priority: 11.07.2022 US 202263388143 P
(43) Date of publication of application: 21.05.2025
(73) Proprietor: Clauson Creative Engineering, Reno, NV 89521 (US)
(72) Inventor: CLAUSON, Luke, W., Reno, NV 89521 (US); SIMMS, James, J., Medford, NJ 08055 (US); REESE, Brendan, C., Reno, NV 89521 (US)
(74) Representative: Slingsby Partners LLP
(86) International application number: PCT/US2023/027267
(87) International publication number: WO 2024/015299

(56) References cited:
- EP-B1- 2 398 433
- WO-A1-2012/136752
- WO-A1-2020/227210
- WO-A1-2021/026538

## Description

### CROSS-REFERENCE TO RELATED APPLICATIONS

This application claims the benefit of priority under 35 U.S.C. §119(e) to copending U.S. Provisional Patent Application Serial No. 63/388,143, filed July 11, 2022.

### BACKGROUND

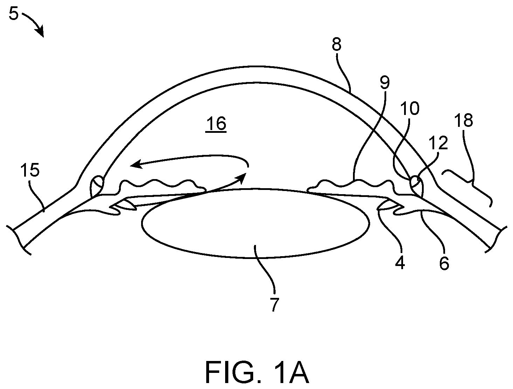

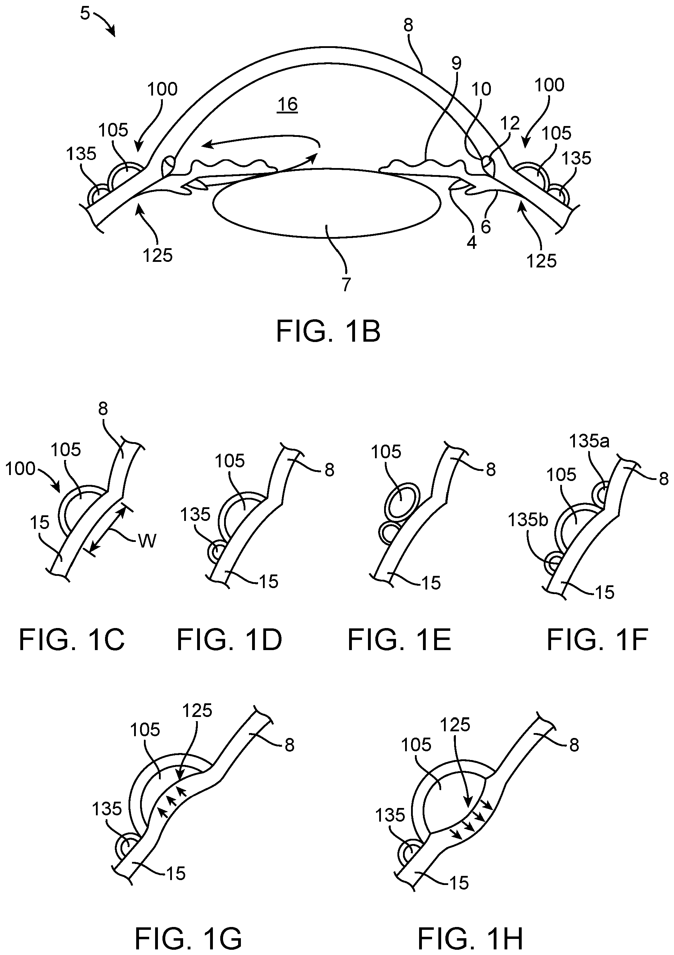

Glaucoma is a complicated disease in which damage to the optic nerve leads to progressive vision loss and is the leading cause of irreversible blindness. Aqueous humor is the fluid that fills the anterior chamber in front of the iris and the posterior chamber of the eye behind the iris. Vitreous humor or vitreous body is a gel-like material found in the posterior segment of the eye posterior of the capsular bag. FIG. 1 is a diagram of the front portion of an eye 5 showing the lens 7, cornea 8, iris 9, ciliary body 6 including ciliary processes 4, trabecular meshwork 10, and Schlemm's canal 12. The aqueous humor is a fluid produced by the ciliary body 6 that lies behind the iris 9 adjacent to the lens 7. This aqueous humor washes over the lens 7 and iris 9 and flows to the drainage system located in the angle of the anterior chamber. The angle of the anterior chamber, which extends circumferentially around the eye, contains structures that allow the aqueous humor to drain.

Some of the aqueous humor is absorbed through the trabecular meshwork 10 into Schlemm's canal 12 into collector channels and passing through the sclera 15 into the episcleral venous circulation. The trabecular meshwork 10 extends circumferentially around the anterior chamber 16 in the angle. The trabecular meshwork 10 limits the outflow of aqueous humor. Schlemm's canal 12 is located beyond the trabecular meshwork 10. The two arrows in the anterior chamber 16 of FIG. 1 show the flow of aqueous humor from the ciliary body 6, over the lens 7, over the iris 9, through the trabecular meshwork 10, and into Schlemm's canal 12 and its collector channels.

In some cases glaucoma is caused by blockage of aqueous humor outflow such as by sclerosis of the trabecular meshwork, pigment or membrane in the angle. In other cases, blockage is due to a closure of the angle between the iris and the cornea. This angle type of glaucoma is referred to as "angle-closure glaucoma". In the majority of glaucoma cases, however, called "open angle glaucoma", the cause is unknown.

Treatments of glaucoma attempt to lower intraocular pressure (IOP) pharmacologically or by surgical intervention that enhance outflow of aqueous humor through the outflow pathways. *Ab externo* trabeculectomy is a type of glaucoma surgery that creates a new path as a "controlled" leak for fluid inside the eye to drain out. Conventionally, a partial thickness scleral flap is formed followed by the creation of a small hole into the anterior chamber. Aqueous humor can flow into the subconjunctival space creating a filtering bleb. The scleral flap is raised up and a blade used to enter the anterior chamber. During the operation a hole is created under the scleral flap that is fluidically connected to the anterior chamber creating an opening. The opening is partially covered with the scleral flap. A small conjunctival "bleb" or bubble appears over the scleral flap, often near the junction of the cornea and the sclera (limbus).

Minimally-invasive surgical procedures provide IOP lowering by enhancing the natural drainage pathways of the eye with minimal tissue disruption. Minimally-invasive glaucoma surgery (MIGS) uses microscopic-sized equipment and tiny incisions. MIGS offers an alternative to conventional glaucoma surgeries with the potential benefit of reducing a patient's dependence on topical glaucoma medication. Trabeculectomies and trabeculotomies can each be performed *ab interno,* or from inside the anterior chamber. *Ab interno* approaches aim to decrease IOP by increasing aqueous humor outflow through a direct opening in the trabecular meshwork from within the anterior chamber so that there is direct communication between the anterior chamber and the outer wall of Schlemm's canal. *Ab interno* approaches include the TRABECTOME (MST / NeoMedix Corp.) electrosurgical instrument that ablates and removes trabecular meshwork, the Kahook Dual Blade (New World Medical) for excisional goniotomy removing a strip of trabecular meshwork, gonioscopy assisted transluminal trabeculotomy (GATT) involving cutting through the trabecular meshwork, cannulating Schlemm's canal, and Omni (Sight Sciences) for performing viscoplasty or trabeculotomy through an *ab interno* approach for cannulating Schlemm's canal. Other *ab interno* methods include the iStent (Glaukos) to create pathway through the trabecular meshwork for improved outflow of aqueous humor through Schlemm's canal.

During these MIGS procedures it is advantageous to be able to visualize the drainage pathways of the eye (e.g., collector channel network). Vital dyes, such as trypan blue, is injected into the Schlemm's Canal and assists in visualization of the fluid flow through the eye when a surgeon at the time of surgery palpates the sclera. The depression of the sclera followed by release causes fluid to be pushed out and refilled. During refilling of the portion of the sclera palpated, trypan blue flows into the drainage pathways, which can be visualized through the sclera. Absence of the color would indicate where a blockage is.

EP 2 398 433 B1 discloses a device for treating an ocular pathology by applying ultrasound. The device comprises an external annular flange suitable to be applied onto the eye globe.

In view of the foregoing, there is a need for improved device related to ophthalmic surgery for the treatment of glaucoma.

### SUMMARY

The ophthalmic device for use on an anterior surface of an eye according to the invention is defined in claim 1. Embodiments of the invention are defined in the dependent claims.

In an aspect, described is an ophthalmic device for use on an anterior surface of an eye. The device includes a treatment chamber defined at least in part by an anterior ring, a posterior ring, and an upper surface connecting the anterior ring and the posterior ring. The treatment chamber is configured to transmit a force against the anterior surface of the eye. The device includes a fixation chamber configured to be in fluid communication with a vacuum source and in fluid communication with the anterior surface of the eye.

The vacuum source can be configured to generate a negative pressure within the fixation chamber to maintain the device against the anterior surface of the eye during transmitting the force via the treatment chamber. The treatment chamber can include one or more dividers creating a plurality of treatment chambers. The one or more dividers can extend radially relative to a center of the device or circumferentially around the center of the device. The device can further include a light source. The light source can be a separate accessory configured to couple to the device or embedded within a region of the device. The light source can be configured to illuminate a region of the eye via the device. The light source can be configured for photobiomodulation of a region of the eye via the device. The light source can be a red, green, or blue light source. The device can further incorporate a tonometer configured to measure intraocular pressure during a procedure in real-time.

The force transmitted against the anterior surface of the eye by the treatment chamber can be configured to maximize an effect of a therapy. The treatment chamber can be coupled to a vacuum source configured to generate a negative pressure and/or a positive pressure within the treatment chamber so as to transmit the force against the anterior surface of the eye. The treatment chamber can be coupled to a source of vibration configured to generate a vibration within the treatment chamber so as to transmit the force against the anterior surface of the eye. The source of vibration can be a vibrating element embedded within a region of the device. The vibrating element can be a haptic engine, a voice coil/speaker, a piezo actuator, a ribbon vibrator. The device can further include an exciter or transducer configured to vibrate the eye in addition to transmitting the force by the treatment chamber. The treatment chamber may have no lower surface so as to transmit the force by means of a fluid interface. The fluid interface can be a gas interface or a liquid interface. The treatment chamber can have a lower surface so as to transmit the force by means of a mechanical interface. The mechanical interface can include a ribbon transducer forming the lower surface of the treatment chamber.

The device can further include an anterior enclosure extending anteriorly relative to the treatment chamber and the fixation chamber and sized to fully enclose the cornea of the eye upon positioning the treatment chamber against the anterior surface of the eye. The anterior enclosure can be in a form of an eye cup. At least the posterior ring of the treatment chamber can form a seal with the anterior surface of the eye to contain a liquid within the anterior enclosure. The anterior surface of the eye can be the sclera of the eye with eyelids open or the outside surface of the eyelid with eyelids closed.

The force transmitted against the anterior surface of the eye can include vibration provided by a vibration power source. The vibration power source can use monotonic frequencies, harmonic frequencies, random frequencies, white noise, pink noise, blue noise, or brown noise. The vibration power source can use an additive set of integer order harmonics or a superimposed set of non-harmonic frequencies. The vibration power source can use any noise characterized by a power spectral density per unit of bandwidth proportional to 1/f^n, wherein f is a frequency of a signal and n is a real valued number. The vibration power source can be tuned to avoid resonant frequencies of the eye or other frequencies that cause unintended damage to the eye. At least the upper surface of the treatment chamber can be transparent or translucent allowing for visualization of the eye through the treatment chamber.

In an interrelated aspect of the present disclosure, provided is a method of visualizing fluid channels of an eye during an intraocular procedure. The method includes injecting an amount of a dye so the dye flows into a region of an eye; positioning an annular device on an anterior surface of the eye so as to encircle the cornea. At least a portion of the annular device is transparent or translucent in order to visualize at least a portion of the anterior surface under the annular device. The method includes transmitting a positive pressure through at least a region of the annular device to create an inwardly compressive force against the anterior surface of the eye, the inwardly compressive force sufficient to prevent dye from entering a region of the eye near the anterior surface of the eye; and visualizing through the transparent or translucent portion of the annular device a return of the dye into the region of the eye.

In an interrelated aspect of the present disclosure, provided is a method of treating glaucoma of an eye having a cornea and an anterior surface surrounding the cornea including positioning an annular device on the anterior surface of the eye so as to encircle the cornea. The annular device includes a treatment chamber and a fixation chamber transmitting a force to at least a portion of the anterior surface of the eye through the treatment chamber of the annular device, the force sufficient to cause a deformation of the anterior surface of the eye in an inwardly direction, an outwardly direction, or a combination of the inwardly and outwardly directions. The method includes fixing the annular device to the anterior surface of the eye by transmitting a negative pressure through the fixation chamber drawing the fixation chamber against the anterior surface of the eye.

In an interrelated aspect of the present disclosure, provided is a method of treating glaucoma of an eye having a cornea and an anterior surface surrounding the cornea including positioning an annular device on the anterior surface of the eye so as to encircle the cornea. The annular device includes a treatment chamber and a fixation chamber. The method further includes transmitting a vibration to at least a portion of the anterior surface with the treatment chamber; and fixing the annular device to the anterior surface of the eye by transmitting a negative pressure through the fixation chamber drawing the fixation chamber against the anterior surface of the eye.

In some variations, one or more of the following can optionally be included in any feasible combination in the above methods, apparatus, devices, and systems. More details are set forth in the accompanying drawings and the description below. Other features and advantages will be apparent from the description and drawings.

### BRIEF DESCRIPTION OF THE DRAWINGS

These and other aspects will now be described in detail with reference to the following drawings. Generally, the figures are not to scale in absolute terms or comparatively, but are intended to be illustrative. Also, relative placement of features and elements may be modified for the purpose of illustrative clarity.
FIG. 1A is a diagram of the anterior portion of the eye in cross-section;
FIG. 1B is a diagram of the anterior portion of the eye in cross-section having an implementation of a device positioned over it;
FIG. 1C is a schematic of a single chamber device for treatment and fixation;
FIG. 1D is a schematic of a dual chamber device having a partially tubular treatment chamber and a partially tubular fixation chamber;
FIG. 1E is a schematic of a dual chamber device having a fully tubular treatment chamber and a partially tubular fixation chamber;
FIG. 1F is a schematic of a three-chamber device having partially tubular treatment chamber and two partially tubular fixation chambers;
FIG. 1G is a schematic of the device of FIG. 1D outwardly distending the limbus by application of negative pressure through the treatment chamber;
FIG. 1H is a schematic of the device of FIG. 1D inwardly compressing the limbus by application of positive pressure through the treatment chamber;
FIG. 2A is an anterior view of an implementation of a treatment device;
FIG. 2B is a side cross-sectional view of the device of FIG. 2A;
FIG. 2C is a side view of the device of FIG. 2A positioned on an anterior surface of an eye in cross-section;
FIG. 2D is a detail cross-sectional view of the device in FIG. 2C taken at circle D;
FIG. 3A is an anterior view of another implementation of a treatment device;
FIG. 3B is a perspective view of the device of FIG. 3A;
FIG. 3C is a posterior view of the device of FIG. 3A;
FIG. 3D is a side view of the device of FIG. 3A;
FIG. 3E is a cross-sectional view of the device of FIG. 3D taken along line D-D;
FIG. 4A is an anterior view of another implementation of a device;
FIG. 4B is a perspective view of the device of FIG. 4A;
FIG. 4C is a posterior view of the device of FIG. 4A;
FIG. 4D is a side view of the device of FIG. 4A;
FIG. 4E is a cross-sectional view of the device of FIG. 4D taken along line E-E;
FIG. 5A is a box diagram of an implementation of a treatment system incorporating a control unit having first and second vacuum sources fluidly connected to an annular-shaped device;
FIG. 5B is a box diagram of another implementation of a treatment system incorporating a control unit electrically connected and fluidly connected to an annular-shaped device.

It should be appreciated that the drawings are for example only and are not meant to be to scale. It is to be understood that devices described herein may include features not necessarily depicted in each figure.

### DETAILED DESCRIPTION

Described herein are devices configured to be placed on the anterior surface of the eye, for example during MIGS procedures, to apply a force against the anterior surface of the eye. In some implementations, the device is configured to apply a force to emulate the manual palpation a surgeon might perform on the anterior surface of the eye to aid in visualizing fluid flow through the active and functional collector channels or other fluid flow pathways from the anterior chamber prior to, during, and/or after surgical procedures in the eye, such as trabeculotomy or other MIGS procedures in which tracking fluid flow in the eye is desired. The forces can include inwardly deforming or compressive forces such as by the application of a positive pressure against the surface of the eye. The forces can alternatively or additionally include outwardly deforming or distending forces such as by the application of negative pressure against the surface of the eye. In still other implementations, the force can include vibration of tissues. The forces can be applied directly by a mechanical element contacting the surface of the eye or can be applied hydraulically or pneumatically.

The forces applied to the eye by the devices described herein are sufficient to distend and/or compress the underlying tissue of the eye without significantly increasing intraocular pressure (IOP) in the process. The inwardly and/or outwardly deforming forces can have a therapeutic effect on IOP, such as by remobilizing and stretching tissue of the collection channel network, Schlemm's canal, and/or trabecular meshwork thereby improving outflow of aqueous from the eye and reducing IOP. Without limiting this disclosure to any particular theory or mode of operation of the treatment being provided by the systems described herein, open angle glaucoma is believed to be brought on, at least in part, by the trabecular meshwork collapsing or clogging or otherwise reducing the outflow of aqueous fluid from the anterior chamber through Schlemm's canal. The forces applied by the devices and systems described herein are useful in the treatment of glaucoma by unclogging or reversing the collapse of the trabecular meshwork allowing more aqueous to flow out from the anterior chamber. The forces applied by the devices and systems described herein are also useful in the treatment of glaucoma such as by increasing the permeability of the trabecular meshwork, Schlemm's canal, and/or collector channels and collector channel ostia. The spaces within these outflow pathways can be opened up and flow improved by manually altering their shape and/or by vibrating the tissues to improve and aid in the efficiency of drainage by removing small particles within the trabecular spaces such as pigments, cell debris, fibrin, etc.

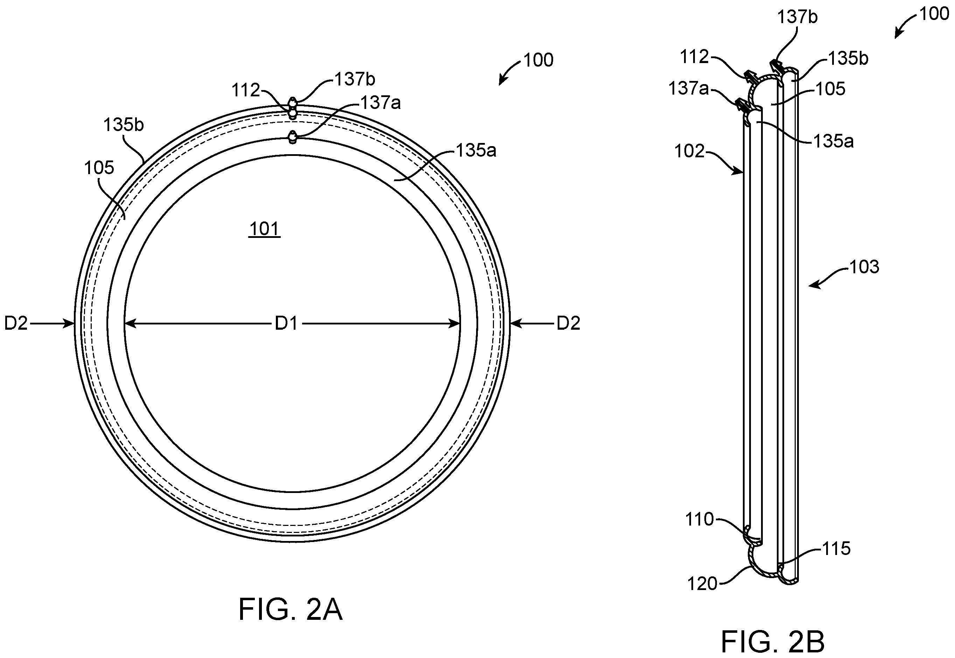

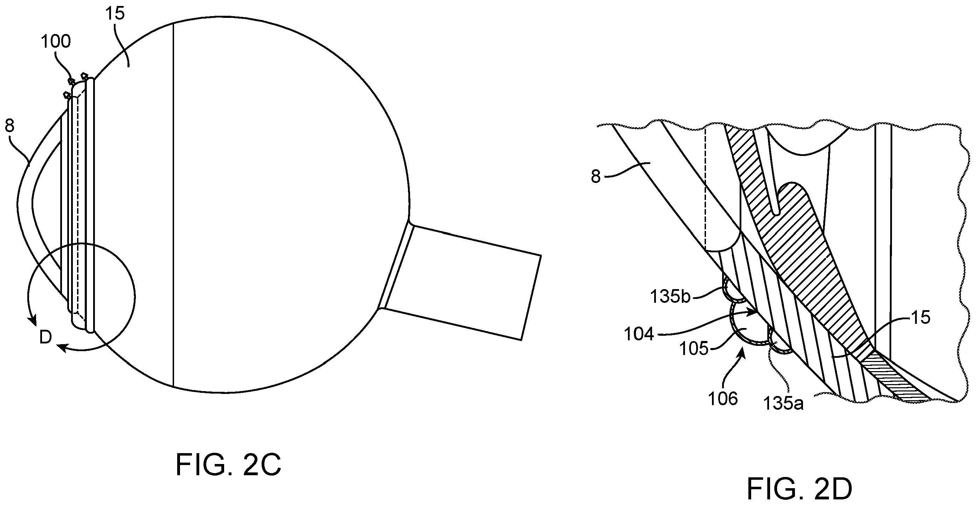

FIGs. 2A-2D illustrate an implementation of the device 100. FIG. 2A is an anterior view, FIG. 2B is a side cross-sectional view, FIG. 2C is a side view of the device positioned on an anterior surface of an eye, and FIG. 2D is a detail view of the device in FIG. 2C taken at circle D. The device 100 can be in the shape of an annulus or a frustum so as to be applied to a treatment region 125 of the eye that can be in the shape of a circumferential band of the globe of an eye. The device 100 can include a central aperture 101 having a first opening at an anterior end 102 of the device 100 and a second opening at a posterior end 103 of the device 100. The first opening has a first inner diameter D1 and the second opening has a second, larger inner diameter D2. The larger inner diameter D2 allows for the posterior end 103 of the device 100 to sit further posterior on the globe of the eye compared to the smaller inner diameter D1 at the anterior end 102 of the device 100, which is configured to sit closer to the cornea 8. The smaller inner diameter D1 can be sized to encircle the cornea 8. The smaller inner diameter D1 may also be sized to sit on the cornea at least in part. D1 can be about 12 mm to about 16 mm and D2 can be about 16 mm to about 20 mm.

FIG. 2D shows an external surface 106 of the device 100, which can be substantially smooth and/or rounded in cross-sectional shape to form a smooth atraumatic surface. The overall shape formed by the internal surface 104 of the device 100 can substantially match and/or conform to a radius of curvature of the anterior surface of the eye so as to be received on the surface of the eye as shown in FIGs. 2C-2D with the treatment region 125 under and/or in contact with the internal surface 104 the device. The treatment region 125 of the eye is preferably near or over a location of the trabecular meshwork, Schlemm's canal, and/or collector channels from the canal. The cross-sectional height of the device 100 from internal surface 104 to external surface 106 can be in a range of about 1 mm to about 10 mm. The width of the device 100 can be at least as wide as the limbal region of the eye, or at least about 1.5 mm - 2.5 mm wide, so that upon placement against the eye, at least a center region of the band of the annulus overlays the limbus 8. The contact width can be at least 1.5 mm up to about 5 mm.

Again with respect to FIGs. 2A-2D, the device 100 includes or forms a treatment chamber 105 configured to transmit a force against the treatment region 125 of the eye by applying a positive pressure and/or a negative pressure, vibration, and the like. In some implementations, the treatment chamber 105 can be formed, in part, by an anterior ring 110, a posterior ring 115 spaced a distance away from the anterior ring 110, and an upper surface 120 that spans between the anterior and posterior rings 110, 115 (see FIG. 2B). The upper surface 120 forms an upper surface to the treatment chamber 105 and the anterior and posterior rings 110, 115 form side surfaces to the treatment chamber 105. The treatment chamber 105 can, but need not have a lower surface against the eye. Meaning, that in some implementations, the treatment chamber 105 is defined by a fully tubular structure (see FIG. 1E) and in others the treatment chamber 105 is defined by a partially tubular structure, for example, so that the treatment chamber 105 is semi-circular or c-shaped as in FIG. 2B and also FIGs. 1C, 1D, 1F, 1G, 1H. The partially tubular treatment chamber 105 can become completely enclosed upon application of the device 100 to the anterior surface of the eye so that the anterior surface of the eye forms the lower surface of the treatment chamber 105 such as illustrated in FIG. 2D and also FIGs. 1C, 1D, 1F, 1G, and 1H.

The anterior ring 110 can extend 360 degrees around the device 100 and have an inner diameter that is at least 10 mm so as to substantially encircle the cornea 8. In some implementations, the inner diameter of the device is sufficient to avoid contact with the cornea 8, such as at least about 12 mm. The inner diameter of the anterior ring 110 is preferably no greater than about 13 mm so that the device 100 upon positioning on the sclera ensures the treatment chamber 105 is capable of overlaying at least a portion of the limbus 18. The posterior ring 115 also extends 360 degrees around the device and is spaced away from the inner ring 110 by a distance of about 4 mm to about 8 mm for a device 100 in which D1 is about 12-16mm and D2 is about 16-20 mm. As such, the posterior ring 115 has an inner diameter that is at least about 14 mm and preferably no greater than about 20 mm. The anterior ring 110 can define the inner diameter at the anterior end of the device and the posterior ring 115 can define the inner diameter at the posterior end of the device. Alternatively, the device 100 incorporates one or more additional rings other than the rings 110, 115 that define the treatment chamber 105. The additional rings can define other chambers of the device 100, such as one or more fixation chambers 135, which will be described in more detail below.

In the implementation of a partially tubular treatment chamber 105, the lower surfaces of the anterior ring 110 and the posterior ring 115 make contact with corresponding surfaces of the eye so as to enclose a treatment region 125 under the treatment chamber 105 (see FIGs. 1B, 1G, 1H). In the implementation of a fully tubular treatment chamber 105, the lower surface of the chamber 105 makes contact with corresponding surface of the eye so as to cover the treatment region 125 under the treatment chamber 105 (see FIG. 1E). The treatment region 125 can include the Schlemm's canal and trabecular meshwork. The treatment region 125 preferably includes the limbus 18 of the eye, which is the band around the eye that forms the border between the transparent cornea 8 and the opaque sclera 15. Underlying tissues within the eye, such as Schlemm's canal and the trabecular meshwork, under the area of the limbus are also incorporated by the treatment region 125 (see FIG. 1A).

The devices are described herein as having a ring shape and configured to encircle the cornea 8. It should be appreciated that the device need not avoid making contact with the cornea. For example, the fixation chamber can be sufficiently low durometer and atraumatic such that the device 100 can contact the cornea 8. In this implementation, the inner diameter D1 of the device can be less than 10 mm such that the device lies on at least some part of the cornea. The device 100 also need not be in the shape of a ring (D1 = 0 mm) so as to cover the cornea entirely, such as like a contact lens. In other implementations, the device 100 has an overall configuration and shape of an eye cup. The eye cup-shaped device 100 can incorporate a posterior end having annular-shaped treatment section as described above and an anterior enclosure sized to extend anterior to the cornea 8 to fully enclose the cornea 8. The eye cup-shaped device has no central aperture 101 that extends through the full thickness of the device 100 *(i.e.,* no opening on the anterior end 102). The anterior enclosure can create a fluid interface for the surface of the eye with the eyelids open *(i.e.,* the sclera and cornea combined) or the outside surface of the eyelid with the eyelids closed. The eye cup-shaped device can be configured contain a fluid within the enclosure. At least a portion of the annular-shaped device 100 applied to the anterior surface of the eye at a location posterior to the treatment region 125 can seal with the eye so as to prevent the fluid within the cup from seeping out. In some implementations, a negative pressure applied through the treatment chamber 105 or another annular chamber of the device can form the seal between the eye and the device 100 to allow for the liquid within the eye cup (*e.g.,* balanced saline solution) from leaking. The liquid within the eye cup can provide cooling, if necessary, as well as moisture to the cornea during treatment of the eye. Any of a variety of aqueous media can be used within the eye cup as known in the art.

The device 100 can be formed of one or more thermoplastic elastomers, such as silicone elastomers. The material of the device 100 forming the fixation chamber(s) 115 can be 30 Shore A - 90 Shore A durometer. The material of the device 100 forming the treatment chamber(s) 105 can be 30 Shore A - 90 Shore D durometer. The material is selected so as to maintain a desired shape even when the treatment chamber 105 is placed under negative or positive pressure. A limiting factor of the amount of pressure applied through the chambers of the device is the eye and amount of distension achieved without causing an increase in IOP. Preferably, the pressure applied through the treatment and fixation chambers is -5 mmHg to about -50 mm Hg. The upper surface 120 is preferably able to maintain its shape through the treatment chamber 105 without collapsing upon transmission of a negative pressure to the treatment chamber 105 or ballooning outward upon transmission of a positive pressure to the treatment chamber 105. The material and/or cross-sectional thickness of the upper surface 120 can be selected to prevent distortion and/or collapse of the treatment chamber during application of a force. In some implementations, the device 100 can incorporate one or more reinforcements within the material to improve retention of its shape during application of a treatment through the treatment chamber 105. For example, the device 100 can be formed of a first material such as silicone elastomer and reinforced in one or more locations with a more rigid material, such as a metal or a more rigid plastic, to provide hoop strength or resistance to the first material. The device 100 can be injection molded or otherwise molded into a selected shape to form the various chambers and rings of the device.

At least some portion of the device 100 can be transparent or translucent to aid in the visualization of the underlying treatment region 125. For example, the upper surface 120 forming the upper surface of the treatment chamber 105 (and the lower surface of the treatment chamber 105, if fully tubular) can be a translucent or transparent material, such as polycarbonate, polyvinyl chloride (PVC), ethylene vinyl acetate (EVA), and the like, allowing the user to see the treatment region 125 underneath the treatment chamber 105, for example, to visualize flow paths of the eye and the eye structures infused by trypan blue or another vital dye. The dye will highlight flow paths until application of a compressive force against the treatment region 125. The compressive force on the eye prevents the dye from flowing through the flow paths and disappears from view. Upon release of the compressive force, the dye returns to the area once again highlighting those paths. The transparent material of the device 100 allows for a user to see the dye return through the flow paths after a period of force is applied by the treatment chamber against the treatment region 125.

In the above implementation, the force applied by the device 100 is an inwardly deforming or compressive force and can be achieved by delivering a positive pressure through the treatment chamber 105. The upper surface 120 can be sufficiently rigid that when a positive pressure is delivered through the treatment chamber 105, the upper surface 120 avoids deforming outward and instead the pressure is urged against the surface of the eye under the treatment chamber 105 to compress the tissue downward *(i.e.,* inward relative to the globe of the eye). The force applied via the treatment chamber 105 can also include an inwardly deforming force and can be achieved by delivering a negative fluid pressure through the treatment chamber 105. The upper surface 120 can be sufficiently rigid that when a negative pressure is delivered through the treatment chamber 105, the upper surface 120 avoids collapse inward towards the eye and instead the pressure urges the surface of the eye under the treatment chamber 105 to outwardly deform the tissue *(i.e.,* outward relative to the globe of the eye). The force applied via the treatment chamber 105 can also include alternative inwardly and outwardly deforming forces via alternating negative and positive fluid pressure through the treatment chamber 105.

The force applied against the treatment region 125 can also include direct contact of a material against the treatment region 125 as opposed to changes in hydraulic or pneumatic pressure. The force can be applied by the internal surface 104 of the device 100. For example, instead of a fluid interface formed by the treatment chamber 105 that applies the force against the treatment region 125, the treatment chamber 105 can be fully tubular such that an internal surface 104 of the device can be a solid component that interfaces with the treatment region 125 of the eye *(i.e.,* either directly on the sclera or indirectly on the sclera through the eyelid) to apply the force against the eye. In some implementations, the interface to apply the force against the eye is a solid interface such as a ribbon transducer. In other implementations, the mechanical force is applied by a vibration element, which will be described in more detail below.

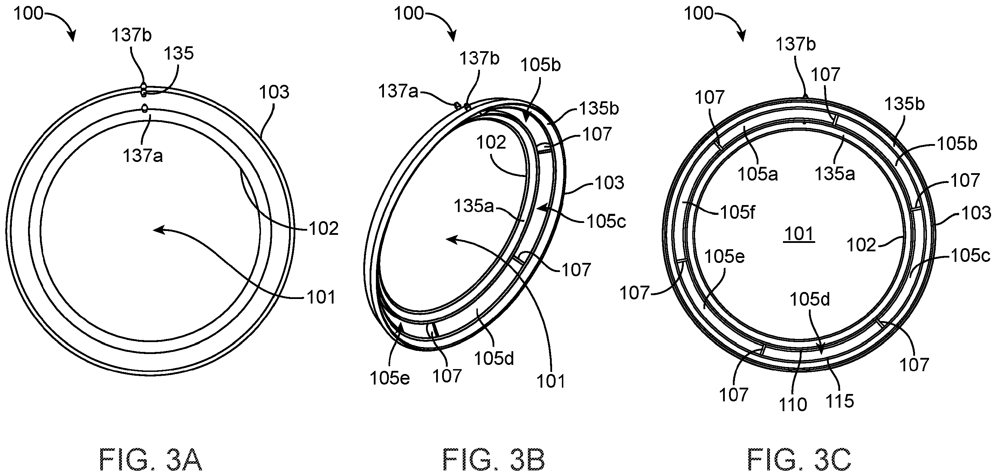

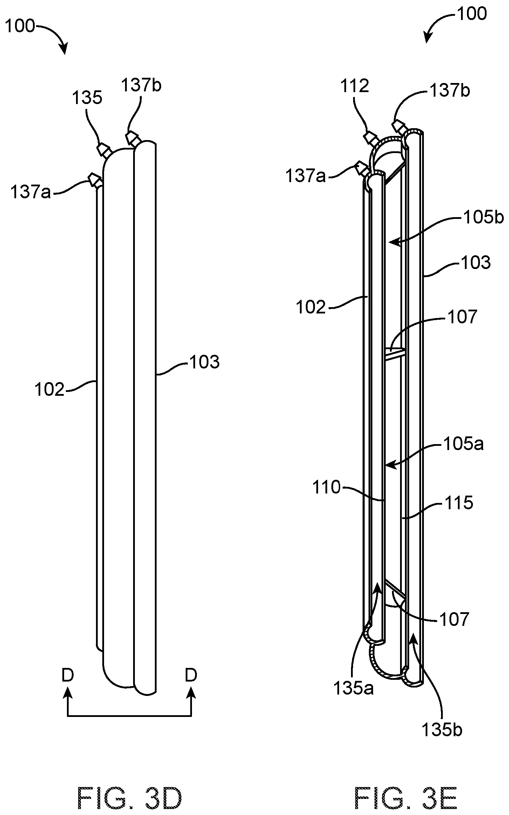

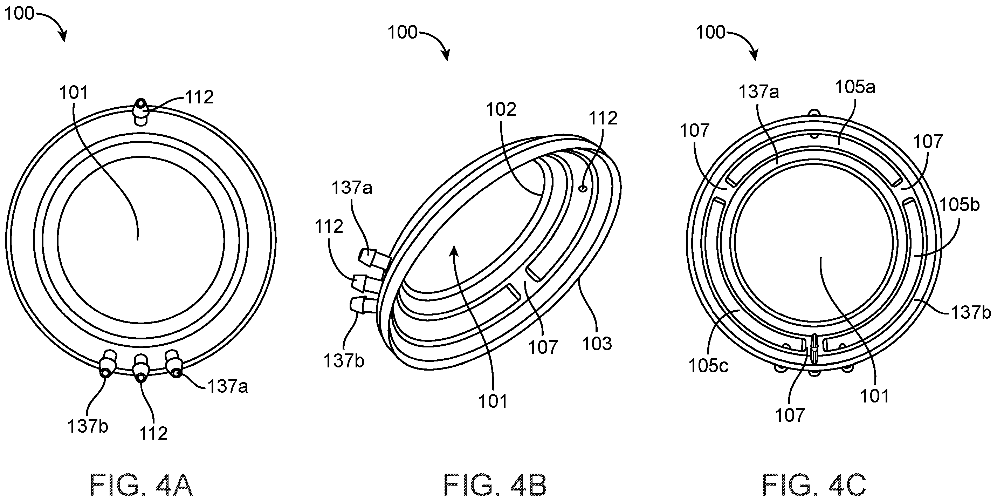

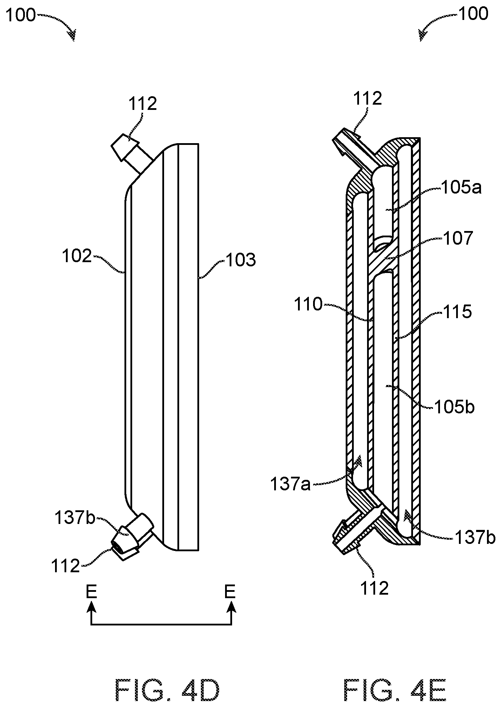

Again with respect to FIGs. 2A-2D, the device 100 can incorporate one or more fixation chambers 135 configured to affix the device 100 to the anterior surface of the eye via negative pressure delivered through the fixation chamber 135. The fixation chamber(s) 135 can extend a full circumference of the device anterior to and/or posterior to the treatment chamber 105. The fixation chamber(s) 135 may also extend only a limited portion around the circumference of the device. FIGs. 2A-2D and also FIGs. 3A-3E, and FIGs. 4A-4E illustrate three-chamber versions of the device 100 having a primary treatment chamber 105 encircled by a first fixation chamber 135a on an anterior end 102 and a second fixation chamber 135b on a posterior end 103 of the treatment chamber 105. FIG. 1F also illustrates a two fixation chambers 135 in addition to the treatment chamber(s) 105. FIGs. 1B, 1D, 1E, 1G, 1H each illustrate implementations of a device with a single fixation chamber 135. In still further implementations, no separate fixation chamber 135 is incorporated and vacuum is applied through the primary treatment chamber 105 sufficient to fix the device to the eye (see FIG. 1C, for example).

The fixation chambers 135 can be partially tubular in cross-sectional shape so that negative pressure applied to the fixation chambers 135 results in the device 100 being sucked down onto the anterior surface of the eye for fixation of the device 100 to the eye. FIG. 2B illustrates the first and second fixation chambers 135a, 135b encircling both sides of the treatment chamber 105 a full circumference of the device 100. Each of the fixation chambers 135a, 135b as well as the treatment chamber 105 is partially tubular and has a c-shape cross-section. The anterior ring 110 of the treatment chamber 105 can be shared with and form a posterior wall for the fixation chamber 135a. Similarly, the posterior ring 115 of the treatment chamber 105 can be shared with and form an anterior wall for the fixation chamber 135b.

Each of the fixation chambers 135 and also the treatment chamber 105 of the device 100 can incorporate a corresponding fitting designed to communicate negative and/or positive pressure from one or more pressure sources. The treatment chamber 105 can include a barb fitting 112 and each of the fixation chambers 135 can incorporate a corresponding fitting 137. FIGs. 2A-2B shows a first fitting 137a that communicates with a first fixation chamber 135a and a second fitting 137b that communicates with a second fixation chamber 137b. The fittings 112, 137 can be barb fittings designed to mate with a segment tubing that connects to a negative or positive pressure source, which will be described in more detail below.

The treatment chamber 105 of the device 100 can extend a full circumference of the device as shown in FIGs. 2A-2D, such that the corresponding treatment region 125 extends a full circumference of a band of the eye. Alternatively, the treatment chamber 105 can be divided into two or more treatment chambers 105 by radially extending dividers 107 (see FIGs. 3A-3E, and FIGs. 4A-4E). The radially extending dividers 107 create multiple treatment chambers 105 each extending less than a circumference of the device 100 so that the treatment region 125 also extends less than a full circumference of a band of the eye. For example, the device 100 can incorporate two radially extending dividers 107 creating two treatment chambers 105 that extend about 180 degrees each around the circumference of the device. The device 100 can incorporate three radially extending dividers 107 creating three treatment chambers 105 that extend about 120 degrees each around the circumference of the device 100. With each additionally divider 107, more treatment chambers 105 can be created that extend shorter distances around the circumference of the device 100. FIG. 3C shows an implementation of a device 100 including six radially extending dividers 107 that create a corresponding number of treatment chambers 105a, 105b, 105c, 105d, 105e, and 105f that each extend about 60 degrees around the circumference of the device 100. FIG. 4C shows an implementation of a device 100 including 3 radially extending devices 107 that create a corresponding number of treatment chambers 105a, 105b, and 105c. The dividers 107 can extend circumferentially around the device so as to create more than one concentric treatment chambers 105 that each extend a full circumference of the device 100. In this implementation, the dividers 107 are ring shaped and each of the multiple treatment chambers 105 extend 360 degrees around the device 100. Each treatment chamber 105, whether created by radially extending dividers 107 or circumferentially extending dividers 107, can incorporate a corresponding fitting 112 so as to connect the separate treatment chambers 105 to a pressure source. The treatment region 125 treated by the treatment chamber(s) 105 of the device 100 can be annular or semi-annular or another shape. The multiple treatment chambers 105, whether fully annular in shape or extending only along a limited arc of the device 100, can be used independently to apply pressure/vacuum at specific locations around the perimeter of the limbus 18. The pressure within each treatment chamber 105 can be changed independently to create higher and lower pressure chambers. For example, the treatment chambers 105 can be activated according to a particular sequence (e.g., inward, outward, random) to apply a massaging force against the anterior surface of the eye. As an example, the treatment chambers 105 are in a concentric arrangement. The outer concentric treatment chamber 105 can be first activated to apply a force against the eye. The outer concentric treatment chamber 105 can then be deactivated and the first inner concentric treatment chamber 105 activated to apply a force. The first inner concentric treatment chamber 105 can be deactivated and the next inner concentric treatment chamber 105 can be activated to apply a force, and so on. Once each of the treatment chambers is activated in turn, the sequence can begin again with the outer treatment chamber or can begin a sequence back out towards the outer chamber sequentially activating each concentric chamber in particular order. The treatment chambers 105 can be filled with a liquid and act as a hydraulic treatment chamber or can be air-filled and act as a pneumatic treatment chamber. The pneumatic/hydraulic treatment frequency range can be about 1 Hz up to about 5000 Hz.

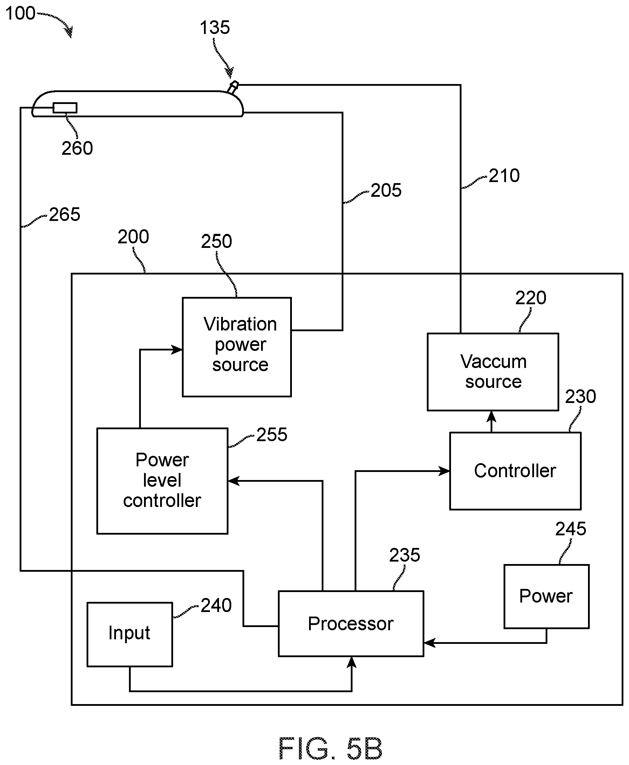

The treatment chamber 105 need not be connected to a pressure source. In an implementation, the device 100 can incorporate an exciter or transducer designed to vibrate the eye alone or in addition to the pressure source designed to apply a force via the primary treatment chamber 105. The vibration can be applied by the device 100 via a vibrating element embedded within the polymer, such as a haptic engine, voice coil/speaker, piezo actuator, ribbon vibrator, plastic muscle (electrically deformable plastic) or other element that is designed to create a vibration within the device 100. Where the treatment chamber 105 is configured to apply vibration force, the chamber 105 can be coupled to a line configured to generate vibration by the vibrating element. The line can electronically couple the vibrating element within the device 100 to a control unit 200 configured to energize the vibrating element electronically to vibrate (see FIG. 5B), which will be described in more detail below.

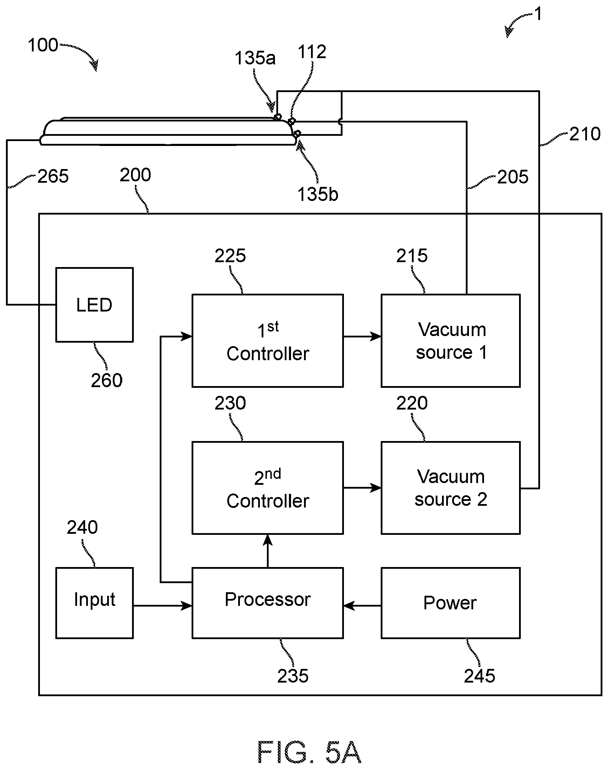

FIG. 5A illustrates an implementation of a system 1 for treatment of an eye including a device 100 for positioning onto and applying a treatment to a region of the eye and a control unit 200. The device 100 is configured to be fluidly coupled to the control unit 200 configured to generate a force within the treatment chamber 105 via treatment line 205. The treatment line 205 fluidly connects the treatment chamber 105 of the device 100 to a first vacuum source 215, which can be a treatment pump configured to be actively operated to apply a particular treatment regimen, within the control unit 200. The vacuum source 215 can also be a passive source of negative pressure, such as a syringe, that is then actively controlled to achieve a particular treatment regimen, such as by valving or other mechanism to affect the application of the vacuum provided by the syringe. The first vacuum source 215 can be controlled by the control unit 200 to generate a force within the treatment chamber 105. The control unit 200 can include a fast-switching valve that operates within a desired range to achieve the massaging force, such as in a range of 1 Hz - 5000 Hz, 100 Hz - 2500 Hz, or about 1000 Hz.

The system 1 is also configured to affix the device 100 to the eye via fixation line 210, which fluidly connects the fixation chamber(s) 135 of the device 100 to a second vacuum source 220, which can be a fixation pump, within the control unit 200. The force transmitted by the treatment chamber 105 can be an outwardly deforming or distending force provided by a negative pressure (*i.e.,* less than atmospheric pressure) generated by the first vacuum source 215 and transmitted through the treatment line 205 to the treatment chamber 105. The force transmitted by the treatment chamber 105 can be an inwardly deforming or compressive force provided by a positive pressure (*i.e.,* greater than atmospheric pressure) generated by the first vacuum source 215 and transmitted through the treatment line 205 to the treatment chamber 105. The force transmitted within the treatment chamber 105 can be a combination of an inwardly or outwardly deforming force provided by alternating negative and positive pressures within the treatment chamber 105. The force transmitted within the treatment chamber 105 can be a combination of an outwardly deforming force and release of the outwardly deforming force provided by alternating a negative pressure with a release of the negative pressure back towards atmospheric. The force transmitted within the treatment chamber 105 can be a combination of an inwardly deforming or compressive force and release of the inwardly deforming or compressive force provided by alternating a positive pressure with a release of the positive pressure back towards atmospheric pressure. Any of a variety of combinations of pressures can be applied to the treatment chamber 105 as well as be described in more detail below. The pressures generated by the first vacuum source 215 can vary including about -5 mmHg down to about -50 mmHg so long as that pressure applied avoids increasing IOP, preferably in the range of about -10 to about -30 mmHg. The second vacuum source 220 transmits negative pressure through the fixation line 210 to the fixation chamber(s) 135. The negative pressure generated by the second vacuum source 220 can vary as well, but like the pressures generated for the first vacuum source 215 is preferably in the range of about -10 down to about -30 mmHg.

Still with regard to FIG. 5A, the pressures applied to the treatment chamber 105 can be generated by the first vacuum source 215 and transmitted through the treatment line 205. The treatment line 205 can be a section of tubing configured to attach to an appropriate coupling on the control unit 200 and the corresponding barb fitting 112 on the device 100. The pressures applied to the fixation chamber(s) 135 can be generated by the second vacuum source 220 and transmitted through the fixation line 210. The fixation line 210 can be a section of tubing configured to attach to a coupling on the control unit 200 and the corresponding barb fitting 137 on the device 100.

The control unit 200 can include a first controller 225 in communication with the first vacuum source 215 and a second controller 230 in communication with the second vacuum source 220. Alternatively, a single controller 225/230 of the control unit 200 can be in communication with both the first vacuum source 215 and the second vacuum source 220. The controller(s) 225/230 can be controlled by a processor 235 configured to receive input from an operator on an input device 240. The input device 240 can vary as is known in the art including one or more keypads, buttons, sliders, dials, touchscreens, and the like that are configured to adjust one or more functions of the control unit 200. The control unit 200 can additionally incorporate one or more outputs such as a display, one or more lights, audio speakers, and the like. The control unit 200 can be powered by a power source 245, which can supply power to the vacuum sources via the processor 235 and controller(s) 225/230.

FIG. 5B illustrates another implementation of a system 1 for treatment of an eye including a device 100 for positioning onto and applying a treatment to a region of the eye. The device 100 is configured to be electronically coupled to the control unit 200 via treatment line 205 and fluidly coupled via fixation line 210. The fixation line 210 connects the fixation chamber 135 of the device 100 to a vacuum source 220 within the control unit 200. In some implementations, the device 100 includes more than a single fixation chamber 135 with each chamber 135 incorporating a corresponding barb fitting 137. The fixation line 210 can have a Y-connector configured to split the line 210 into corresponding tube segments that are configured to connect to the barb fittings 137 so as to transmit negative pressure to each of the fixation chambers 135. The vacuum source 220 can be controlled by the control unit 200 to generate a negative pressure within the fixation chamber 135. The negative pressure transmitted through the fixation chamber 135 can be sufficient to affix the device onto the eye via suction force so that the device 100 remains in place during application of force (e.g., negative pressure, positive pressure, vibration, etc.) through the treatment chamber 105. The vacuum source 220 can include a controller 230 that is controlled by processor 235 that is configured to receive input from an operator on input device 240. The control unit 200 is powered by a power source 245, which supplies power to the vacuum source 220 via the processor 235 and controller 230.

Still with respect to FIG. 5B, the device 100 can incorporate a vibrating element embedded within a region of the device to apply vibration to the eye via the treatment chamber 105. The vibration element is connected to the control unit via treatment line 205, which electronically couples the vibration element to a vibration power source 250. The processor 235 provides control outputs to the power level controller 255, which provides a signal to the vibration power source 250. The signal and power delivered depends upon the type of vibrating element within the device 100.

The vibration power source 250 can use monotonic frequencies or harmonic frequencies. The vibration power source 250 can use random frequencies, white noise, or other sonic hues (*i.e.* pink, blue, brown, etc.). The noise can be any noise characterized by a power spectral density per unit of bandwith proportional to 1/f^n, where f is the frequency of the signal and n is a real valued number. The vibration power source 250 can use an additive set of integer order harmonics or a superimposed set of non-harmonic (e.g., non-integer multiple frequencies). The frequency can be impedance-matched to effectively transmit the pressure, suction, and/or vibration to the target tissues of the eye (e.g., trabecular meshwork and associated fluid channels) while minimizing reflection of the pressure, suction, or vibration. In order to impedance match the acoustic properties of the acoustic transmitter with the target tissue, a specific membrane, fluid, or gel may be used. The interface has an appropriate density and speed of sound such that acoustic energy is more efficiently coupled with the target tissue with less of the acoustic energy being reflected. This same principle may be used when acoustic impedance matching gel is applied during ultrasound examination. The gel reduces acoustic reflection and facilitates more efficient transmission of ultrasonic energy into the tissue. The vibration power source 250 is preferably tuned to avoid resonant frequencies of the eye or other frequencies that may cause damage to eye structures.

Ultrasound is known to break calcification and stenosis. In some implementations, the device 100 can incorporate low frequency pulsing with the addition of trans-scleral ultrasound to aid in breaking up stenosis such as in Schlemm's canal, trabecular meshwork, and the ostia of collector channels. In this implementation the vibration power source 250 is an ultrasound power source that is configured to transmit higher frequencies in the ultrasound range (e.g., greater than 20 kHz, up to about 2 - 18 megahertz) to the vibrating element embedded within the device. The vibrating element (e.g., piezoelectric crystal) can be embedded within a region of the device 100 and coupled electrically via line 205 to the vibration power source 250 to transmit high frequencies to the eye. The energy, whether ultrasound or other vibration frequency, is applied in an annular treatment area 125 of the eye. The power source 250 can be a sine-wave signal generator at a determined frequency between about 1 kHz and 18 MHz. The power source can also combine various frequencies in the range of 1 KHz to 18 MHz to create a plurality of combinations as described above. The vibration power source 250 can use random frequencies, white noise, or other sonic hues as described herein. The noise can be any noise characterized by a power spectral density per unit of bandwidth proportional to 1/f^n, wherein f is the frequency of the signal and n is a real valued number. The vibration power source 250 can use an additive set of integer order harmonics or a superimposed set of non-harmonic (e.g., non-integer multiple frequencies).

The system 1 can incorporate one or more light sources 260 to illuminate structures of the eye while in use. In one implementation, the light source 260 is an LED within the control unit 200 that is transmitted to a region of the device 100 via a fiber optic, light pipe or other sort of linkage 265 (see FIG. 5A). In another implementation, the light source 260 is an LED embedded within a region of the device 100 that is powered via an electrical connection 265 from the control unit 200 (see FIG. 5B). In still further implementations, the light source 260 can be a separate accessory that is used in combination with the device 100 and the control unit 200 such as a light accessory that is placed over a region of the device 100 to transmit light towards a region of the eye. The light source 260 can be used in conjunction with the pressurization or application of vacuum to the surface of the eye to aid in the visualization of the fluid flow during the procedure.

The one or more lights sources 260 can be configured for visualization and/or photobiomodulation. For example, one or more light sources 260 can be LEDs or laser diodes and corresponding fiber optics incorporated within the device or the control unit 200 to perform photobiomodulation including red (600-700 nm), near-infrared (770-1200 nm), white, blue, green, ultraviolet or near ultraviolet, or other colors.

The system 1 or the device 100 can incorporate a tonometer configured to measure intraocular pressure (IOP) during a procedure in real-time. In an implementation, the device 100 can incorporate a bridge or other feature extending inward from an anterior end 102 of the device 100 within the inner diameter of the central aperture 101 to position a sensor near or against the cornea 8 when the device 100 is positioned against the sclera 15. The sensor can take readings periodically throughout the treatment period and communicate the data to the control unit 200 via an electrical linkage. The tonometer can be a non-contact tonometer configured to apply air pressure against the cornea with a brief puff of air. The tonometer can also be a contact sensor that comes in direct contact with the front of the eye.

The device 100 can be positioned on a surface of the eyeball directly as illustrated *(i.e.,* the sclera with the eyelids open) or can be a surface of the eyelid with the eyelid closed over the sclera 15. In some implementations, the device 100 can be coupled to or used in combination with an eye speculum configured to hold open the eyelids during use. For example, an outer perimeter of the device 100 can incorporate one or more clips biased to spring outward to urge the upper and lower eyelids away from one another.

### METHODS OF USE

The devices described herein can be used to aid in visualizing fluid channels of an eye, for example, during an intraocular procedure. The devices described herein can be used for palpating the eye along a full circumferential band so as to visualize flow of vital dyes such as Trypan blue for assessing outflow during a procedure. The devices described herein can be used during glaucoma treatment by fixating to the eye using suction and performing a treatment on the treatment region, such as by applying suction, positive pressure, a combination of suction and positive pressure, vibration, and the like according to a treatment frequency while the device is affixed to a region of the eye by suction.

In an implementation, the device is used for visualizing fluid channels of an eye during an intraocular procedure. An amount of a dye, such as Trypan blue, is injected locally so that the dye enters into a region of an eye. The device is positioned on an anterior surface of the eye. At least a portion of the device is transparent or translucent so as to visualize at least a portion of the anterior surface of the eye underlying the annular device. A positive pressure is transmitted through at least a region of the annular device to create an inwardly compressive force against the anterior surface of the eye. The inwardly compressive force is sufficient to prevent the dye from entering a region of the eye near the anterior surface of the eye. A surgeon visualizes through the transparent or translucent portion of the device a return of the eye into a region of the eye upon release of the positive pressure. The device can be an annular device sized to encircle at least a portion of the cornea. The inwardly compressive force to prevent the dye from enter the region of the eye can vary, but is preferably sufficient to substantially prevent fluid flow through the channels in the region being compressed similar to how a surgeon may palpate an eye with a finger during a procedure. The force can be applied to a circumferential band of an eye or along one or more distinct regions of an eye as described elsewhere herein. The device can additionally incorporate a chamber configured to affix the device to the eye during use. The fixation suction can be about -5 mmHg, preferably about -10 mmHg. The fixation suction can be no greater than about -50 mmHg, preferably no greater than about -30 mmHg.

**In** another implementation, the device is used as a method of treating glaucoma of an eye. The device is positioned on the anterior surface of the eye so as to encircle at least a portion of the cornea. The device includes a treatment chamber and a fixation chamber. A force is transmitted to at least a portion of the anterior surface of the eye through the treatment chamber of the device. The force is sufficient to cause a deformation of the anterior surface of the eye in an inwardly direction, an outwardly direction, or a combination of the inwardly and outwardly directions. The device is fixed to the anterior surface of the eye by transmitting a negative pressure through the fixation chamber drawing the fixation chamber against the anterior surface of the eye. The device can be an annular device sized to encircle at least a portion of the cornea. The pneumatic/hydraulic treatment frequency range can be about 1 Hz - 5000 Hz. The fixation suction can be about -5 mmHg, preferably about - 10 mmHg. The fixation suction can be no greater than about -50 mmHg, preferably no greater than about -30 mmHg.

In another implementation, the device is used as a method of treating glaucoma of an eye. The device is positioned on the anterior surface of the eye and includes a treatment chamber and a fixation chamber. The device transmits a vibration with the treatment chamber to at least a portion of the anterior surface of the eye. The device is fixed to the anterior surface of the eye by transmitting a negative pressure through the fixation chamber drawing the fixation chamber against the anterior surface of the eye. The device can be an annular device sized to encircle at least a portion of the cornea. The piezo or other electrical treatment frequency range can be about 1 kHz - 18 MHz. The fixation suction can be about - 5 mmHg, preferably about -10 mmHg. The fixation suction can be no greater than about -50 mmHg, preferably no greater than about -30 mmHg.

The system can include a control unit, power source, microprocessor computer, and the like. Aspects of the subject matter described herein may be realized in digital electronic circuitry, integrated circuitry, specially designed ASICs (application specific integrated circuits), computer hardware, firmware, software, and/or combinations thereof. These various implementations may include an implementation in one or more computer programs that are executable and/or interpretable on a programmable system including at least one programmable processor, which may be special or general purpose, coupled to receive signals, data and instructions from, and to transmit signals, data, and instructions to, a storage system, at least one input device, and at least one output device.

These computer programs (also known as programs, software, software applications, or code) include machine instructions for a programmable processor, and may be implemented in a high-level procedural and/or object-oriented programming language, and/or in assembly/machine language. As used herein, the term "machine-readable medium" refers to any computer program product, apparatus, and/or device (e.g., magnetic discs, optical disks, memory, Programmable Logic Devices (PLDs)) used to provide machine instructions and/or data to a programmable processor, including a machine-readable medium that receives machine instructions as a machine-readable signal. The term "machine-readable signal" refers to any signal used to provide machine instructions and/or data to a programmable processor.

In various implementations, description is made with reference to the figures. However, certain implementations may be practiced without one or more of these specific details, or in combination with other known methods and configurations. In the description, numerous specific details are set forth, such as specific configurations, dimensions, and processes, in order to provide a thorough understanding of the implementations. In other instances, well-known processes and manufacturing techniques have not been described in particular detail in order to not unnecessarily obscure the description. Reference throughout this specification to "one embodiment," "an embodiment," "one implementation, "an implementation," or the like, means that a particular feature, structure, configuration, or characteristic described is included in at least one embodiment or implementation. Thus, the appearance of the phrase "one embodiment," "an embodiment," "one implementation, "an implementation," or the like, in various places throughout this specification are not necessarily referring to the same embodiment or implementation. Furthermore, the particular features, structures, configurations, or characteristics may be combined in any suitable manner in one or more implementations.

The use of relative terms throughout the description may denote a relative position or direction. For example, "distal" may indicate a first direction away from a reference point. Similarly, "proximal" may indicate a location in a second direction opposite to the first direction. The reference point used herein may be the operator such that the terms "proximal" and "distal" are in reference to an operator using the device. A region of the device that is closer to an operator may be described herein as "proximal" and a region of the device that is further away from an operator may be described herein as "distal". Similarly, the terms "proximal" and "distal" may also be used herein to refer to anatomical locations of a patient from the perspective of an operator or from the perspective of an entry point or along a path of insertion from the entry point of the system. A region of the device that is designed to be arranged on an eye in an anterior direction or towards a front part of the eye may be described herein as "anterior" and a region of the device that is further away from the front part of the eye towards the back of the eye may be described herein as "posterior". As such, a location that is proximal may mean a location in the patient that is closer to an entry point of the device along a path of insertion towards a target and a location that is distal may mean a location in a patient that is further away from an entry point of the device along a path of insertion towards the target location. However, such terms are provided to establish relative frames of reference, and are not intended to limit the use or orientation of the devices to a specific configuration described in the various implementations.

As used herein, the term "about" means a range of values including the specified value, which a person of ordinary skill in the art would consider reasonably similar to the specified value. In aspects, about means within a standard deviation using measurements generally acceptable in the art. In aspects, about means a range extending to +/- 10% of the specified value. In aspects, about includes the specified value.

While this specification contains many specifics, these should not be construed as limitations on the scope of what is claimed or of what may be claimed, but rather as descriptions of features specific to particular embodiments. Certain features that are described in this specification in the context of separate embodiments can also be implemented in combination in a single embodiment. Conversely, various features that are described in the context of a single embodiment can also be implemented in multiple embodiments separately or in any suitable sub-combination. Moreover, although features may be described above as acting in certain combinations and even initially claimed as such, one or more features from a claimed combination can in some cases be excised from the combination, and the claimed combination may be directed to a sub-combination or a variation of a sub-combination. Similarly, while operations are depicted in the drawings in a particular order, this should not be understood as requiring that such operations be performed in the particular order shown or in sequential order, or that all illustrated operations be performed, to achieve desirable results. Only a few examples and implementations are disclosed. Variations, modifications and enhancements to the described examples and implementations and other implementations may be made based on what is disclosed.

In the descriptions above and in the claims, phrases such as "at least one of" or "one or more of" may occur followed by a conjunctive list of elements or features. The term "and/or" may also occur in a list of two or more elements or features. Unless otherwise implicitly or explicitly contradicted by the context in which it is used, such a phrase is intended to mean any of the listed elements or features individually or any of the recited elements or features in combination with any of the other recited elements or features. For example, the phrases "at least one of A and B;" "one or more of A and B;" and "A and/or B" are each intended to mean "A alone, B alone, or A and B together." A similar interpretation is also intended for lists including three or more items. For example, the phrases "at least one of A, B, and C;" "one or more of A, B, and C;" and "A, B, and/or C" are each intended to mean "A alone, B alone, C alone, A and B together, A and C together, B and C together, or A and B and C together."

Use of the term "based on," above and in the claims is intended to mean, "based at least in part on," such that an unrecited feature or element is also permissible.

The systems disclosed herein may be packaged together in a single package. The finished package would be sterilized using sterilization methods such as Ethylene oxide or radiation and labeled and boxed. Instructions for use may also be provided in-box or through an internet link printed on the label.

All methods described herein can be performed in any suitable order unless otherwise indicated herein or otherwise clearly contradicted by context. No language in the specification should be construed as indicating any non-claimed element essential to the practice of the invention.

## Claims

1. An ophthalmic device (100) for use on an anterior surface of an eye, the device comprising:
a treatment chamber (105) defined at least in part by an anterior ring (110) having an inner diameter sized to encircle at least a cornea of the eye, a posterior ring (115) having an inner diameter sized greater than the inner diameter of the anterior ring, and an upper surface (120) connecting the anterior ring and the posterior ring, wherein a lower surface of the anterior ring and a lower surface of the posterior ring are each arranged to contact the eye while in use so that the treatment chamber (105) transmits a force against a region (125) of the anterior surface of the eye under the treatment chamber, wherein the force is inwardly deforming or compressive, outwardly deforming or distending, or a combination thereof; and
a fixation chamber (135) configured to be in fluid communication with a vacuum source (220) and in fluid communication with the anterior surface of the eye.

2. The ophthalmic device (100) of claim 1, wherein the vacuum source (215) is configured to generate a negative pressure within the fixation chamber (135) to maintain the device against the anterior surface of the eye during transmitting the force via the treatment chamber (125).

3. The ophthalmic device (100) of claim 1 or claim 2, wherein the treatment chamber (125) comprises one or more dividers (107) creating a plurality of treatment chambers, wherein the one or more dividers extend radially relative to a center of the device or circumferentially around the center of the device.

4. The ophthalmic device (100) of any one of the preceding claims, further comprising a light source (260), wherein the light source is a separate accessory configured to couple to the deviceor wherein the light source is embedded within a region of the device.

5. The ophthalmic device (100) of claim 4, wherein the light source (260) is configured to illuminate a region of the eye via the device and/or wherein the light source is configured for photobiomodulation of a region of the eye via the device wherein the light source is a red, green, or blue light source.

6. The ophthalmic device (100) of any one of the preceding claims, further comprising a tonometer configured to measure intraocular pressure during a procedure in real-time.

7. The ophthalmic device (100) of any one of the preceding claims, wherein the treatment chamber (125) is coupled to a further vacuum source (215) configured to generate a negative pressure and/or a positive pressure within the treatment chamber so as to transmit the force against the anterior surface of the eye.

8. The ophthalmic device (100) of any one of the preceding claims, wherein the treatment chamber (105) has no lower surface so as to transmit the force by means of a fluid interface.

9. The ophthalmic device (100) of claim 8, wherein the fluid interface is a gas interface or a liquid interface.

10. The ophthalmic device (100) of any one of claims 1 to 7, where the treatment chamber (105) has a lower surface so as to transmit the force by means of a mechanical interface.

11. The ophthalmic device (100) of claim 10, wherein the mechanical interface comprises a ribbon transducer forming the lower surface of the treatment chamber.

12. The ophthalmic device (100) of any one of the preceding claims, further comprising an anterior enclosure extending anteriorly relative to the treatment chamber and the fixation chamber and sized to fully enclose the cornea of the eye upon positioning the treatment chamber against the anterior surface of the eye.

13. The ophthalmic device (100) of claim 12, wherein the anterior enclosure is in a form of an eye cup, wherein at least the posterior ring of the treatment chamber (105) forms a seal with the anterior surface of the eye to contain a liquid within the anterior enclosure.

14. The ophthalmic device (100) of any one of the preceding claims, wherein at least the upper surface of the treatment chamber (105) is transparent or translucent allowing for visualization of the eye through the treatment chamber.

## Patentansprüche

1. Ophthalmologische Vorrichtung (100) zur Anwendung an der vorderen Augenoberfläche, die Vorrichtung umfassend:
eine Behandlungskammer (105), die mindestens teilweise durch einen vorderen Ring (110) mit einem Innendurchmesser, der so bemessen ist, dass er mindestens die Hornhaut des Auges umschließt, einen hinteren Ring (115) mit einem Innendurchmesser, der größer ist als der Innendurchmesser des vorderen Rings, und eine obere Oberfläche (120), die den vorderen Ring und den hinteren Ring verbindet, wobei eine untere Oberfläche des vorderen Rings und eine untere Oberfläche des hinteren Rings jeweils so angeordnet sind, dass sie während des Gebrauchs das Auge berühren, sodass die Behandlungskammer (105) eine Kraft auf einen Bereich (125) der vorderen Oberfläche des Auges unterhalb der Behandlungskammer ausübt, wobei die Kraft nach innen verformend oder komprimierend, nach außen verformend oder dehnend oder eine Kombination davon ist; und
eine Fixierungskammer (135), die konfiguriert ist, um in Fluidverbindung mit einer Vakuumquelle (220) und mit der vorderen Augenoberfläche steht.

2. Ophthalmologische Vorrichtung (100) nach Anspruch 1, wobei die Vakuumquelle (215) konfiguriert ist, um einen Unterdruck innerhalb der Fixierungskammer (135) zu erzeugen, um die Vorrichtung während der Kraftübertragung über die Behandlungskammer (125) an der vorderen Augenoberfläche zu halten.

3. Ophthalmologische Vorrichtung (100) nach Anspruch 1 oder 2, wobei die Behandlungskammer (125) eine oder mehrere Trennwände (107) umfasst, die eine Vielzahl von Behandlungskammern bilden, wobei sich die eine oder mehreren Trennwände radial relativ zu einem Zentrum der Vorrichtung oder in Umfangsrichtung um das Zentrum der Vorrichtung erstrecken.

4. Ophthalmologische Vorrichtung (100) nach einem der vorstehenden Ansprüche, die ferner eine Lichtquelle (260) umfasst, wobei die Lichtquelle ein separates Zubehörteil ist, das konfiguriert ist, um an die Vorrichtung angekoppelt werden kann, oder wobei die Lichtquelle in einen Bereich der Vorrichtung eingebettet ist.

5. Ophthalmologische Vorrichtung (100) nach Anspruch 4, wobei die Lichtquelle (260) konfiguriert ist, um einen Bereich des Auges über die Vorrichtung zu beleuchten, und/oder wobei die Lichtquelle zur Photobiomodulation eines Bereichs des Auges über die Vorrichtung ausgelegt ist, wobei die Lichtquelle eine rote, grüne oder blaue Lichtquelle ist.

6. Ophthalmologische Vorrichtung (100) nach einem der vorstehenden Ansprüche, die ferner ein Tonometer umfasst, das konfiguriert ist, um den Augeninnendruck während eines Eingriffs in Echtzeit zu messen.

7. Ophthalmologische Vorrichtung (100) nach einem der vorstehenden Ansprüche, wobei die Behandlungskammer (125) mit einer weiteren Vakuumquelle (215) verbunden ist, die konfiguriert ist, um innerhalb der Behandlungskammer einen Unterdruck und/oder einen Überdruck zu erzeugen, um die Kraft auf die vordere Augenoberfläche auszuüben.

8. Ophthalmologische Vorrichtung (100) nach einem der vorstehenden Ansprüche, wobei die Behandlungskammer (105) keine untere Oberfläche aufweist, um die Kraft über eine Fluidgrenzfläche zu übertragen.

9. Ophthalmologische Vorrichtung (100) nach Anspruch 8, wobei die Fluidgrenzfläche eine Gasgrenzfläche oder eine Flüssigkeitsgrenzfläche ist.

10. Ophthalmologische Vorrichtung (100) nach einem der Ansprüche 1 bis 7, wobei die Behandlungskammer (105) eine untere Oberfläche aufweist, um die Kraft mittels einer mechanischen Grenzfläche zu übertragen.

11. Ophthalmologische Vorrichtung (100) nach Anspruch 10, wobei die mechanische Grenzfläche einen Bandwandler umfasst, der die untere Oberfläche der Behandlungskammer bildet.

12. Ophthalmologische Vorrichtung (100) nach einem der vorstehenden Ansprüche, die ferner ein vorderes Gehäuse umfasst, das sich relativ zur Behandlungskammer und zur Fixierungskammer nach vorne erstreckt und so dimensioniert ist, dass es die Hornhaut des Auges vollständig umschließt, wenn die Behandlungskammer an der vorderen Augenoberfläche positioniert wird.

13. Ophthalmologische Vorrichtung (100) nach Anspruch 12, wobei das vordere Gehäuse die Form einer Augenmuschel aufweist, wobei mindestens der hintere Ring der Behandlungskammer (105) eine Abdichtung mit der vorderen Augenoberfläche bildet, um eine Flüssigkeit innerhalb des vorderen Gehäuses einzuschließen.

14. Ophthalmologische Vorrichtung (100) nach einem der vorstehenden Ansprüche, wobei mindestens die obere Oberfläche der Behandlungskammer (105) transparent oder durchscheinend ist, sodass das Auge durch die Behandlungskammer hindurch sichtbar ist.

## Revendications

1. Dispositif ophtalmique (100) pour une utilisation sur une surface antérieure d'un œil, le dispositif comprenant :
une chambre de traitement (105) définie au moins en partie par un anneau antérieur (110) ayant un diamètre interne dimensionné pour entourer au moins une cornée de l'œil, un anneau postérieur (115) ayant un diamètre interne dimensionné pour être supérieur au diamètre interne de l'anneau antérieur, et une surface supérieure (120) reliant l'anneau antérieur et l'anneau postérieur, dans lequel une surface inférieure de l'anneau antérieur et une surface inférieure de l'anneau postérieur étant chacune agencée pour entrer en contact avec l'œil pendant l'utilisation de sorte que la chambre de traitement (105) transmet une force contre une région (125) de la surface antérieure de l'œil sous la chambre de traitement, dans lequel la force est une force de déformation ou de compression vers l'intérieur, de déformation ou de distension vers l'extérieur, ou une combinaison de celles-ci ; et
une chambre de fixation (135) configurée pour être en communication fluidique avec une source de vide (220) et en communication fluidique avec la surface antérieure de l'œil.

2. Dispositif ophtalmique (100) selon la revendication 1, dans lequel la source de vide (215) est configurée pour générer une pression négative à l'intérieur de la chambre de fixation (135) afin de maintenir le dispositif contre la surface antérieure de l'œil pendant la transmission de la force via la chambre de traitement (125).

3. Dispositif ophtalmique (100) selon la revendication 1 ou la revendication 2, dans lequel la chambre de traitement (125) comprend un ou plusieurs séparateurs (107) créant une pluralité de chambres de traitement, dans lequel les un ou plusieurs séparateurs s'étendent radialement par rapport à un centre du dispositif ou circonférentiellement autour du centre du dispositif.

4. Dispositif ophtalmique (100) selon l'une quelconque des revendications précédentes, comprenant en outre une source lumineuse (260), dans lequel la source lumineuse est un accessoire distinct configuré pour être couplé au dispositif ou dans lequel la source lumineuse est intégrée à l'intérieur d'une région du dispositif.

5. Dispositif ophtalmique (100) selon la revendication 4, dans lequel la source lumineuse (260) est configurée pour éclairer une région de l'œil via le dispositif et/ou dans lequel la source lumineuse est configurée pour la photobiomodulation d'une région de l'œil via le dispositif, dans lequel la source lumineuse est une source lumineuse rouge, verte ou bleue.

6. Dispositif ophtalmique (100) selon l'une quelconque des revendications précédentes, comprenant en outre un tonomètre configuré pour mesurer la pression intraoculaire pendant une procédure en temps réel.

7. Dispositif ophtalmique (100) selon l'une quelconque des revendications précédentes, dans lequel la chambre de traitement (125) est couplée à une source de vide (215) supplémentaire configurée pour générer une pression négative et/ou une pression positive à l'intérieur de la chambre de traitement de manière à transmettre la force contre la surface antérieure de l'œil.

8. Dispositif ophtalmique (100) selon l'une quelconque des revendications précédentes, dans lequel la chambre de traitement (105) n'a pas de surface inférieure de manière à transmettre la force au moyen d'une interface fluide.

9. Dispositif ophtalmique (100) selon la revendication 8, dans lequel l'interface fluide est une interface gazeuse ou une interface liquide.

10. Dispositif ophtalmique (100) selon l'une quelconque des revendications 1 à 7, dans lequel la chambre de traitement (105) a une surface inférieure de manière à transmettre la force au moyen d'une interface mécanique.

11. Dispositif ophtalmique (100) selon la revendication 10, dans lequel l'interface mécanique comprend un transducteur à ruban formant la surface inférieure de la chambre de traitement.

12. Dispositif ophtalmique (100) selon l'une quelconque des revendications précédentes, comprenant en outre une enceinte antérieure s'étendant antérieurement par rapport à la chambre de traitement et à la chambre de fixation et dimensionnée pour entourer entièrement la cornée de l'œil lors du positionnement de la chambre de traitement contre la surface antérieure de l'œil.

13. Dispositif ophtalmique (100) selon la revendication 12, dans lequel l'enceinte antérieure se présente sous la forme d'une cupule oculaire, dans lequel au moins l'anneau postérieur de la chambre de traitement (105) forme un joint avec la surface antérieure de l'œil afin de contenir un liquide à l'intérieur de l'enceinte antérieure.

14. Dispositif ophtalmique (100) selon l'une quelconque des revendications précédentes, dans lequel au moins la surface supérieure de la chambre de traitement (105) est transparente ou translucide, permettant la visualisation de l'œil à travers la chambre de traitement.
